# EUROPEAN PATENT SPECIFICATION

(11) **EP 0 836 455 B1**
(45) Date of publication and mention of the grant of the patent: **16.04.2003**
(21) Application number: 96917995.1
(22) Date of filing: 06.06.1996
(51) Int. Cl.: A61F 2/44

(54) **TRANSLATERAL SPINAL IMPLANT**
TRANSLATERALE WIRBELSÄULENIMPLANTATIE
ARTHRODESE TRANSLATERAL DU RACHIS

(30) Priority: 07.06.1995 US 479596
(43) Date of publication of application: 22.04.1998
(60) Divisional application: 02027647.3
(73) Proprietor: MICHELSON, Gary Karlin, Venice, CA 90291 (US)
(72) Inventor: MICHELSON, Gary Karlin, Venice, CA 90291 (US)
(74) Representative: Grünecker, Kinkeldey, Stockmair & Schwanhäusser Anwaltssozietät
(86) International application number: US9608612
(87) International publication number: WO96039988

(56) References cited:
- EP-A- 0 419 564
- US-A- 4 328 593
- US-A- 4 834 757
- US-A- 4 878 915
- US-A- 4 911 718
- US-A- 5 263 953

## Description

This invention relates generally to spinal fusion implants, and more particularly to spinal fusion implants for insertion from the side of a patient (translateral) across the transverse width of the spine and between two adjacent vertebrae.

In the past, spinal fusion implants have been inserted only from either an anterior or posterior direction, from the front or the back of the patient. Such implants are well known in the art and may have cylindrical, rectangular and other shapes. In the past, Cloward, Wilterberger, Crock, Viche, Bagby, Brantigan, and others have taught various methods involving the drilling of holes across the disc space between two adjacent vertebrae of the spine for the purpose of causing an interbody spinal fusion. Cloward taught placing a dowel of bone within that drilled hole for the purpose of bridging the defect and to be incorporated into the fusion. Viche taught the threading of that bone dowel. Bagby taught the placing of the bone graft into a metal bucket otherwise smooth on its surface, except for rows of radially placed holes communicative to the interior of the basket and to the bone graft. The Bagby device was disclosed as capable of being used in a horse. Brantigan taught the use of inert blocks preferably made of metal and having that metal at its external surface imitate the porosity of bone. Brantigan theorized that the bone dowel could be replaced entirely with a metal plug that, while not itself active in the fusion, would nevertheless serve to support the vertebrae from within the disc space while allowing fusion to occur around it.

U.S. Patent No. 3,844,601 issued to Ma et al. on November 19, 1974, teaches a method and instrumentation for preparing rectangular spaces across the disc space into the adjacent vertebrae and for preparing a rectangular graft of the bone itself that is inserted in the rectangular spaces.

U.S. Patent No. 4,743,256 issued to Brantigan on May 10, 1988 teaches the use of an inert artificial spacer in the shape of a rectangle in place of using a rectangular bone graft as taught by Ma et al.

U.S. Patent No. 4,878,915 issued to Brantigan on November 7, 1989, teaches the use of fully cylindrical inert implants for use in interbody spinal fusion. Such implants do not participate in the bone fusion process but act as inert spacers and allow for the growth of bone to the outer surfaces of the implants.

U.S. Patent No. 4,834,757 issued to Brantigan on May 30, 1989, teaches a rectangular shaped, hollow spinal fusion implant for use in lieu of a rectangular bone graft or Brantigan's earlier artificial inert spacer.

However, all of the prior implants have been inserted from either the front or the back of the patient. As a result, the spinal fusion implants of the past were necessarily limited in size to the dimensions of the vertebrae relative to the direction in which the implants were inserted. For example, the maximum possible length for an implant that is inserted from either the front or the back of the patient is limited to the depth of the vertebrae, the depth of a vertebrae being the dimension of the vertebrae measured from the anterior end to the posterior end of the vertebrae. It was not previously possible to insert an implant that had a length that was greater than the depth of the vertebrae from front to back as such an implant would protrude from either the anterior or posterior aspect of the spine resulting in great harm to the patient.

In U.S. Patent No. 5,015,247 to Michelson, a cylindrical threaded implant is described for insertion across the disc space between two adjacent vertebrae. Such an implant was disclosed as being inserted either from the front of the patient or from the back and has a diameter larger than the disc space so that it engages both of the adjacent vertebrae.

The maximum diameter possible with a cylindrical implant that is inserted from the front or the back of the patient is limited by at least two factors. The first factor limiting the diameter of a cylindrical implant is realized when an attempt is made to use a single, centrally placed implant from either the front or the back of the patient. Such an implant must be large enough to occupy a sufficient portion of the transverse width of the disc space to promote firm stability. The use of an implant that is placed in the disc space to stabilize the two adjacent vertebrae requires that the vertebrae be stable when the implant is in place, otherwise there will not be any bone bridging between the implant and the vertebrae. If a single implant is used in the center of the disc space, inherent instability is created, as the vertebrae are generally free to rock back and forth over the implant which serves as a fulcrum. However, to achieve the required stability, it would be necessary to use the widest possible implant and the excursion of such a large single implant into the adjacent vertebrae would be so severe that the two vertebrae would be virtually cut in half.

The second factor which limits the diameter size of a cylindrical implant is in the situation where two cylindrical implants are implanted from either the front or the back of the patient and placed side-by-side across a disc space and into the two adjacent vertebrae in an attempt to gain stability while avoiding the problems of the single implant. Such implants require a diameter that is sufficiently large to penetrate into and significantly engage each of the adjacent vertebrae yet the diameter may not be so large that it is no longer possible to place two such implants side-by-side and to still have them contained within the transverse width of the spine.

The use of multiple implants requires that the implants be small enough so as to fit into the same limited spinal width. These implants being of smaller diameter as limited by the need to place more than one within the width of the spine then penetrate only minimally into the depth of the vertebral bone.

Also, the insertion of multiple implants requires multiple procedures, essentially a duplication of any procedure done on one side of the center line must also be performed on the other side of the center line.

It is, therefore, an object of the invention to provide the spinal fusion implant that is inserted from the translateral approach from the spine and that is capable of stabilizing the vertebrae adjacent to such an implant and which is safer to use than implants of the past.

This object is solved by the features of claim 1.

Advantages embodiments of the invention are disclosed by the sub claims.

According to the invention, the spinal fusion implant is inserted from the side of the patient, herein referred to as the translateral approach to the spine. The translateral spinal fusion implant of the present invention is inserted into the spine of the patient across the transverse width of the vertebrae to be fused. A transverse width of the vertebrae is measured from one lateral aspect of the spine to the opposite lateral aspect The depth of the vertebrae is measured from the interior aspect to the posterior aspect of the spine.

The implant is dimensioned to fit within the bore created across the disk space and into the adjacent vertebrae. Such an implant may be substantially cylindrical and has an outer surface comprising a bone engaging means for engaging the implant to the adjacent vertebrae. The translateral spinal fusion implant of the present invention has a length that is greater than one half of the transverse width of the vertebrae and is greater than the depth of the vertebrae. The translateral implant of the present invention has a height that is greater than the height of the disk space between two adjacent vertebrae so as to engage both of the vertebrae. The width of the implant needs to be only slightly less than the depth of the vertebrae themselves.

The upper and lower surface of such an implant may be contoured as to conform to the shape of the disk space and the adjacent vertebral end plate surfaces. According to one embodiment, the implant has opposed arcuate portions for engaging each of the adjacent vertebrae.

A translateral implant of the present invention is safer to use than implants inserted from the front or the back as the aorta and vena cava lie anterior to the spine and the dural sac and nerves posteriorly, all of which structures are simply avoided in the lateral approach.

The translateral spinal fusion implant of the present invention may be inserted into the disk space through a hollow tube which is engaged in the lateral aspect of the spine through a lateral, anterior or anterolateral incision making the procedure safe and simple.

The translateral spinal fusion implant of the present invention may comprise at least in part a fusion promoting and/or bioactive materials for active participation of the implant in the spinal fusion process.

According to the invention, greater stability of the vertebrae being fused is provided and any fail is less likely. Moreover, the spinal fusion implant is more deeply embedded into the adjacent vertebrae.

These and other objects of the present invention will become apparent from a review of the accompanying drawings and the detailed description of the drawings.

### BRIEF DESCRIPTION OF THE DRAWINGS

Figure 1 is a perspective side view of the translateral spinal fusion implant of the present invention having an external thread for engaging the bone of two adjacent vertebrae.
Figure 2 is an elevational view of the anterior aspect of a segment of the spinal column with the spinal fusion implant of Figure 1 inserted from the lateral aspect along the transverse width of the vertebrae.
Figure 3 is an elevational view of the lateral aspect of a segment of the lumbar spine with a first spinal fusion implant of the present invention inserted from the lateral aspect into a hole drilled across a first disc space and into two adjacent vertebrae, and a second spinal fusion implant of the present invention inserted from the lateral aspect into a second hole drilled across a second disc space and into two adjacent vertebrae.
Figure 4 is top sectional view along lines 4--4 of Figure 3 showing the area of contact of the spinal fusion implant of the present invention and the vertebra.
Figure 5 is an anterior elevational view of a segment of the lumbar spine with two cylindrical implants inserted from the anterior of the spine into holes drilled across the same disc space and into two adjacent vertebrae.
Figure 6 is sectional view along lines 6--6 of Figure 5 showing the area of contact between the two implants of Figure 5 and the vertebra.
Figure 7 is a anterior perspective view of a single vertebra and an alternative embodiment of the spinal fusion implant of the present invention in the form of a dowel inserted translaterally into a hole drilled across a disc space and into the vertebra along the transverse width of the vertebra.
Figure 8 is a perspective view of an alternative embodiment of the spinal fusion implant of the present invention having ratchetings for engaging the vertebrae.
Figure 9 is a perspective view of an alternative embodiment of the spinal fusion implant of the present invention having a knurled surface for engaging the vertebrae.
Figure 10 is a perspective view of an alternative embodiment of the spinal fusion implant of the present invention having ratchetings for engaging the vertebrae and a flattened side.
Figure 11 is a perspective view of an alternative embodiment of the spinal fusion implant of the present invention having a knurled surface for engaging the vertebrae and a flattened side.
Figure 12 is a perspective view of an alternative embodiment of the spinal fusion implant of the present invention having a blasted surface for engaging the vertebrae.
Figure 13 is a perspective view of an alternative embodiment of the spinal fusion implant of the present invention having ratchetings for engaging the vertebrae with openings in the form of vertical and horizontal slots.
Figure 14 is a perspective view of an alternative embodiment of the spinal fusion implant of the present invention having longitudinal splines for engaging the vertebrae and openings in the form of vertical slots.
Figure 15 is elevational view of the lateral aspect of the spinal column having the spinal fusion implant of Figure 14 inserted from the lateral aspect along the transverse width of the vertebrae into a hole created across the disc space and into two adjacent vertebrae.
Figure 16 is a perspective side view of an alternative embodiment of the spinal fusion implant of the present invention.
Figure 17 is a elevational anterior view of a segment of the spinal column having the spinal fusion implant of Figure 16 inserted from the lateral aspect in the disc space between two adjacent vertebrae along the transverse width of the vertebrae.
Figure 18 is a perspective side view of an alternative embodiment of the spinal fusion implant of the present invention.
Figure 19 is a perspective lateral anterior view of a segment of the spinal column with a plurality of the spinal implants of Figure 18 shown in hidden line inserted from the lateral aspect in a modular fashion in the disc space between two adjacent vertebrae along the transverse width of the vertebrae.
Figure 20 is perspective view of an alternative embodiment of the spinal fusion implant of the present invention.

### DETAILED DESCRIPTION OF THE DRAWINGS

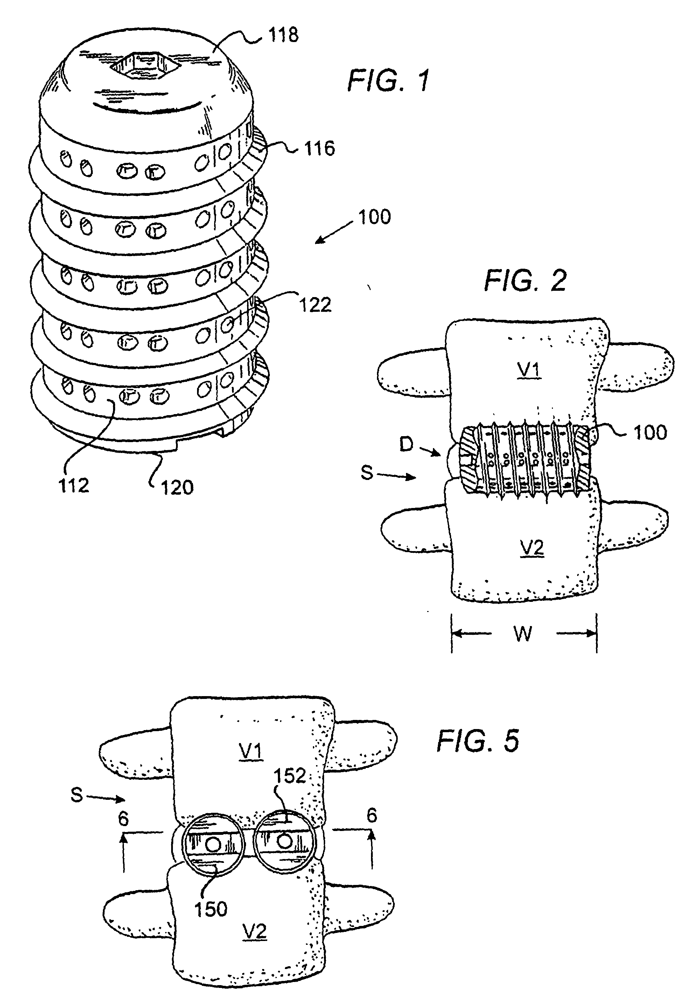

Referring to Figures 1-5, an embodiment of the translateral spinal fusion implant of the present invention, generally referred to by numeral 100, is shown. The spinal fusion implant 100 has a substantially cylindrical configuration having an outer wall 112 surrounding an internal chamber 114 for holding fusion promoting material. The exterior of the spinal fusion implant 100 comprises an external thread 116 suitable for engaging the vertebrae of the spine to stabilize the spinal fusion implant 100 across the disc space and into adjacent vertebrae once surgically implanted. The spinal fusion implant 100 has a removable cap 118 at one end which provides access to the internal chamber 114 and has an insertion end 120 adapted to engage insertion instrumentation.

The cap 118 is removable to provide access to the internal chamber 114, such that the internal chamber 114 can be filled and hold any natural or artificial osteoconductive, osteoinductive, osteogenic, or other fusion enhancing material. Some examples of such materials are bone harvested from the patient, or bone growth inducing material such as, but not limited to, hydroxyapatite, hydroxyapatite tricalcium phosphate; or bone morphogenic protein. The cap 118 and/or the spinal fusion implant 100 itself is made of material appropriate for human implantation such as titanium and/or may be made of, and/or filled and/or coated with a bone ingrowth inducing material such as, but not limited to, hydroxyapatite or hydroxyapatite tricalcium phosphate or any other osteoconductive, osteoinductive, osteogenic, or other fusion enhancing material.

The outer wall 112 comprises openings 122 which may be closed wells or openings communicating into the internal chamber 114 to permit bone ingrowth into the chamber 114.

Referring specifically to Figure 2, an elevational view of the anterior aspect of a segment of the spinal column S with the spinal fusion implant 100 inserted from the lateral aspect of the spinal column S into a hole bored into the adjacent vertebrae V₁ and V₂ across the disc space D. The spinal fusion implant 100 is inserted along the transverse width W of the adjacent vertebrae V₁ and V₂ such that the spinal fusion implant 100 extends translaterally in the direction from one lateral aspect of the vertebrae to the opposite lateral aspect of the vertebrae.

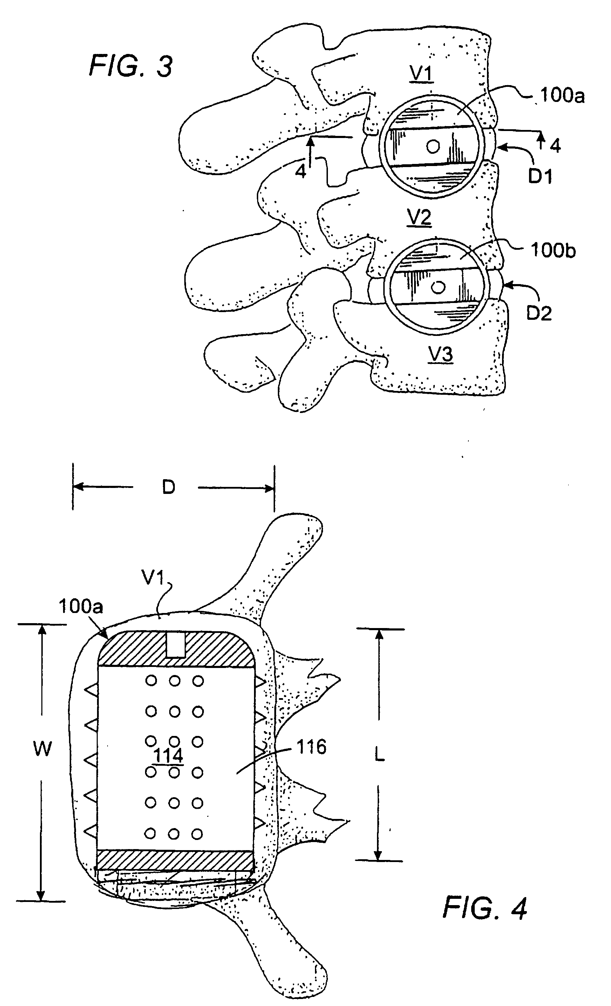

Referring to Figure 3, an elevational view of the lateral aspect of a segment of the lumbar spine S is shown with a first implant 100a, identical to spinal fusion implant 100, inserted from the lateral aspect into a hole bored across a first disc space D₁ and into two adjacent vertebrae V₁ and V₂, and a second implant 100b, identical to spinal fusion implant 100, inserted from the lateral aspect into a second hole bored across a second disc space D₂ and into two adjacent vertebrae V₂ and V₃.

The translateral implants of the present invention are inserted by the translateral method disclosed in copending Application Serial No. 08/394,836 entitled IMPROVED METHODS AND INSTRUMENTATION FOR THE SURGICAL CORRECTION OF HUMAN THORACIC AND LUMBAR SPINAL DISEASE FROM THE LATERAL ASPECT OF THE SPINE, filed on February 27, 1995, which is incorporated herein by reference.

Referring to Figure 4, a top sectional view along lines 4--4 of Figure 3 is shown illustrating the area of contact of the implant 100a and the vertebra V₁. The vertebra V₁ has a depth D measured from the anterior to posterior aspect of the spine, and a transverse width W measured from one lateral aspect to the opposite lateral aspect of the vertebra V₁. The implant 100a has a length L that is substantially greater than the depth D of the vertebra
V₁, such that the implant 100a may extend substantially across the transverse width W of the vertebra V₁. In the preferred embodiment, the implant 100a has a length L that is greater than one half the transverse width W of the vertebrae and has a diameter of a sufficiently large size that approximates the depth D of the vertebra V₁. As a result of the large length and diameter of the implant 100a, a large surface area of contact between the implant 100a and the vertebrae V₁ is possible creating a highly stable construct. The implant 100a has a much greater surface area of contact with the vertebra V₁ than was previously possible with implants that are inserted from the front or the back of the spine.

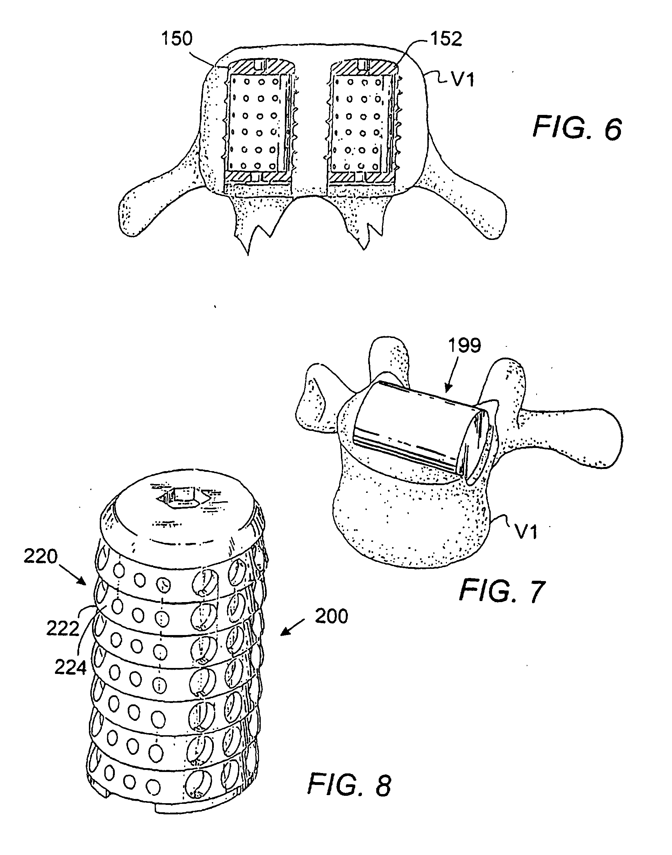

As described above in the Background of the Invention, a centrally placed single implant from either the front or the back of the patient must be large enough to occupy a sufficient portion of the transverse width W of the vertebrae to promote firm stability. However, the vertical height of such an implant and excursion into the adjacent vertebrae would be so severe that if any two consecutive disc spaces were to be operated upon, the vertebra in between the disc spaces would be cut in half. Therefore it has been the practice to use multiple implants, one on each side of the center line (mid-saggital axis) of the vertebrae, thereby providing a greater degree of stability. Referring to Figure 5, an anterior elevational view of a segment of the lumbar spine S is shown with two cylindrical implants 150 and 152 inserted from the anterior aspect of the spine S into holes drilled across the same disc space D and into two adjacent vertebrae V₁ and V₂.

Referring to Figure 6, sectional view along lines 6--6 of Figure 5 illustrating the area of contact between the two implants 150 and 152 inserted from the anterior aspect of the spine and the vertebra V₁ is shown. As can be seen from Figure 6, the surface area of the two spinal implants 150 and 152 in contact with the vertebra V₁ is substantially less than that of a single translateral spinal fusion implant 100 that is inserted across the transverse width W of the vertebra V₁. As a result, a more stable construct is achieved with the translateral spinal fusion implant 100 of the present invention than was previously possible with implants that are inserted from either the front or the back of the patient promoting from stability of the fusion construction.

In the preferred embodiment, the spinal fusion implant 100 of the present invention has an overall length in the range of 35 mm to 50 mm, with 38-44 mm being preferred, and a maximum diameter in the range of 22 mm to 30 mm, with 24-26 mm being preferred when inserted in the lumbar spine. In the thoracic spine such implants would have a length in the range of 12-30 mm, and a maximum diameter in the range of 14-26 mm, with the preferred diameter being 20 mm.

Referring to Figure 7, an anterior perspective view of a single vertebra V₁ and an alternative embodiment of the translateral spinal fusion implant of the present invention, generally referred to by the numeral 199, is shown. The spinal fusion implant 199 is a dowel inserted into a hole drilled across a disc space and into the vertebra V₁ along the transverse width of the vertebra V₁. The spinal fusion implant 199 has the same dimensions as the spinal fusion implant 100 described above. The spinal fusion implant 199 can be made of any material suitable for human implantation may comprise fusion promoting and/or bioactive material to actively participate in the spinal fusion process. The implant 199 can be made of a porous, and/or mesh-like, and/or cancellous material, or any other material suitable for the described purpose.

Referring to Figure 8, an alternative embodiment of the translateral spinal fusion implant of the present invention, is shown and generally referred to by the numeral 200. The spinal fusion implant 200 has a substantially cylindrical configuration having a thin outer wall 212 surrounding an internal chamber 214. The exterior of the spinal fusion implant 200 comprises surface roughenings that provide a surface suitable for engaging the bone of the vertebrae to stabilize the spinal fusion implant 200 across the disc space and into the adjacent vertebrae once surgically implanted. The surface roughenings comprise a plurality of ratchetings 220 along the circumference of the spinal fusion implant 200. Each of the plurality of ratchetings 220 has a bone engaging edge 222 and an angled segment 224.

The spinal fusion implant 200 is implanted into a cylindrical bore derived across the disc space and into two adjacent vertebrae. The spinal fusion implant 200 may be pushed into the cylindrical bore across the disc space by direct, linear advancement since it requires no thread to pull it forward through the spine. As no torque is required to advance the spinal fusion implant 200 there is no minimum requisite height of the surface roughenings.

The ratchetings 220 may face in one direction, the direction in which the spinal fusion implant 200 is inserted, and function to prevent the spinal fusion implant 200 from backing out of the disc space in a direction opposite to the direction of insertion once inserted between the two adjacent vertebrae. The ratchetings 220 urge the spinal fusion implant 200 forward against the unremoved bone of the vertebrae. Since implants generally want to back out along the same path in which they are inserted, the ratchetings 220 tend to urge the spinal fusion implant 200 forward against the solid unremoved bone at the end of the cylindrical bone, further resisting dislodgement and controlling motion resulting in an exceedingly stable implantation.

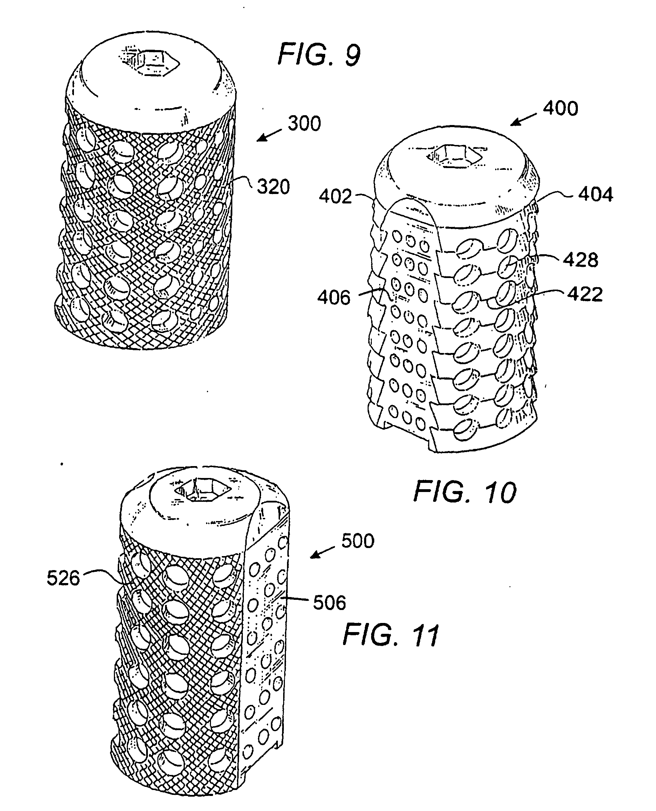

The spinal fusion implant 200 has an engagement means at one end for engaging a driver instrument for intimately engaging and binding the implant 200 and the driver instrument together. Once affixed to the implant driver instrument, the spinal fusion implant 200 may be then introduced through a hollow cylindrical tube and driven into the cylindrical hole that has been drilled across the disc space. The implant driver instrument may then be impacted by a mallet, or similar device, to linearly advance the spinal fusion implant 200 across the disc space. Once the spinal fusion implant 200 is inserted across the disc space, the ratchetings 220, engage the bone of the vertebrae and the implant driver instrument is detached from the spinal fusion implant 200. Referring to Figure 9, an alternative embodiment of the spinal fusion implant of the present invention generally referred to by the numeral 300 is shown. The spinal fusion implant 300 has a substantially cylindrical .configuration having surface roughenings for stabilizing the implant 300 within the intervertebral space D. The surface roughenings comprise a surface knurling 320 such as, but not limited to, the diamond-shaped bone engaging pattern shown in Figure 9. The spinal fusion implant 300 may have surface knurling 320 throughout the entire external surface of the spinal fusion implant 300, throughout only a portion of the external surface, or any combination thereof, without departing from the scope of the present invention. In those circumstances where there is no undrilled bone in the disc space forward of the spinal fusion implant 300 to resist further forward advancement of the implant, surface knurling 320 is preferred as it produces an exceedingly high interference fit with the bone of the vertebrae and resists motion equally in all directions and without the tendency to urge itself forward.

Referring to Figure 10, an alternative embodiment of the spinal fusion implant of the present invention is shown and is generally referred to by the numeral 400. The spinal fusion implant 400 has a similar configuration to that of the spinal fusion implant 200, except that it comprises a partially cylindrical member having arcuate portions 402 and 404 which are arcs of the same circle with portions of its outer wall that are flattened so as to present a first flat side 406. Alternatively, the implant 400 may have a second flat side that is diametrically opposite to the first flat side 406. The spinal fusion implant 400 is substantially the same as the spinal fusion implant 200, except that the openings 428 are positioned on the ratcheting 420 such that the openings 428 are positioned between the bone engaging edges 422 and are not bisected by the bone engaging edges 422.

Referring to Figure 11, an alternative embodiment of the spinal fusion implant of the present invention is shown and generally referred to by the numeral 500. The spinal fusion implant 500 is substantially identical to the spinal fusion implant 400 described above except that in place of ratchetings 420, it has surface knurling 520. The surface knurling 520 assists in the retaining of the spinal fusion implant 500 once it is inserted across the disc space between two adjacent vertebrae. It is recognized that the surface knurling 520 of the implant 500 may be combined with any of a number of other surface roughenings such as, but not limited to, ratchetings to assist in retaining the spinal fusion implant 500 across the disc space.

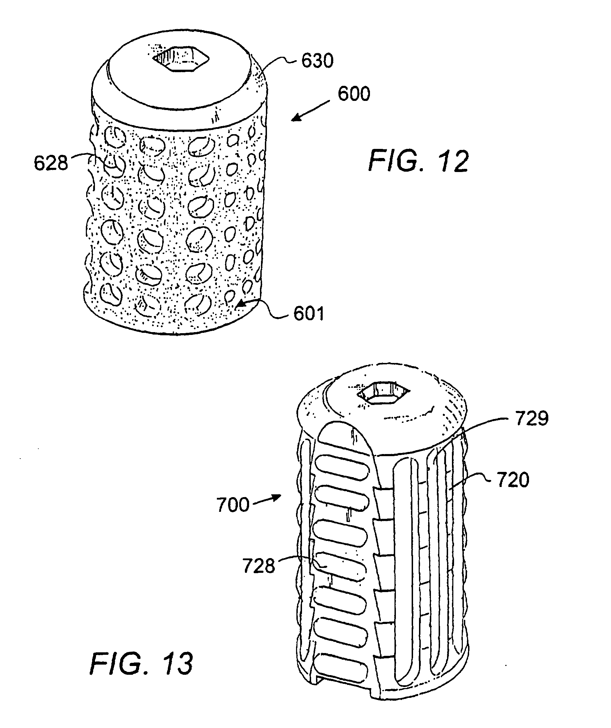

Referring to Figure 12, an alternative embodiment of the spinal fusion implant of the present invention generally referred to by the numeral 600 is shown. The spinal fusion implant 600 has the same structure as the spinal fusion implant 300 described above but instead of knurling 320 has a different surface roughening. The spinal fusion implant 600 has a surface roughening comprising of a blasted external surface 601 which may be stippled to provide an engagement surface for the vertebrae when inserted across the disc space. The spinal fusion implant has a plurality of openings 628, a removable cap 630 for accessing an internal chamber.

Referring to Figure 13, an alternative embodiment of the spinal fusion implant of the present invention generally referred to by the numeral 700 is shown. The spinal fusion implant 700 is similar to spinal fusion implant 400 described above except that it has openings in the form of horizontal slots 728 on the flat side 706 and vertical slots 729 on the cylindrical portion of the spinal fusion implant 700. The spinal fusion implant 700 has ratchetings 720 for engaging the bone of the vertebrae similar to the ratchetings 220 described above.

It is appreciated that the spinal fusion implants of the present invention may include any and all surface roughening configurations that either increase the surface area or interference fit of the implant and the vertebrae. It is appreciated that the ratchetings described above for the various embodiments of the spinal fusion implants of the present invention may also comprise a knurled or other surface roughenings in combination with the ratchetings to further enhance the retention of the spinal fusion implant across the disc space once inserted.

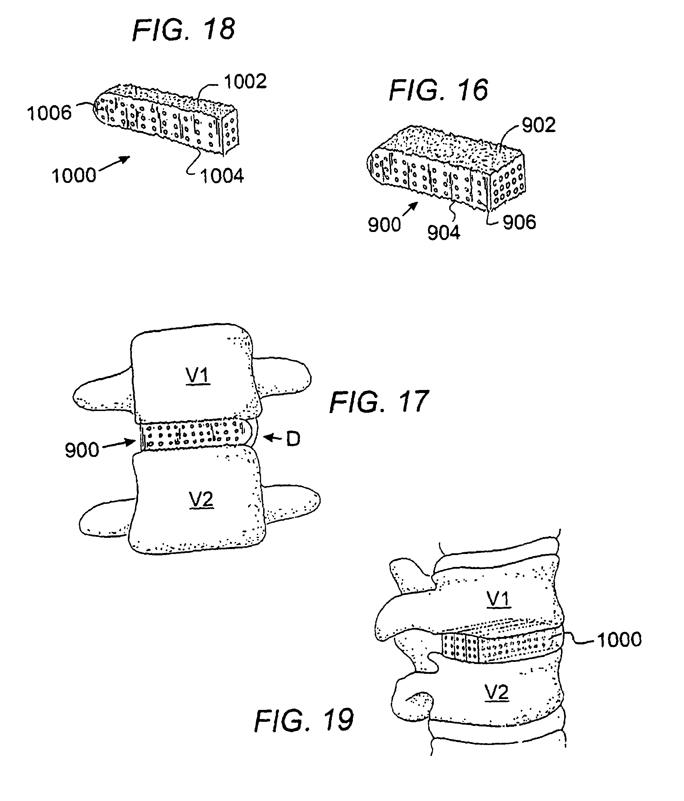

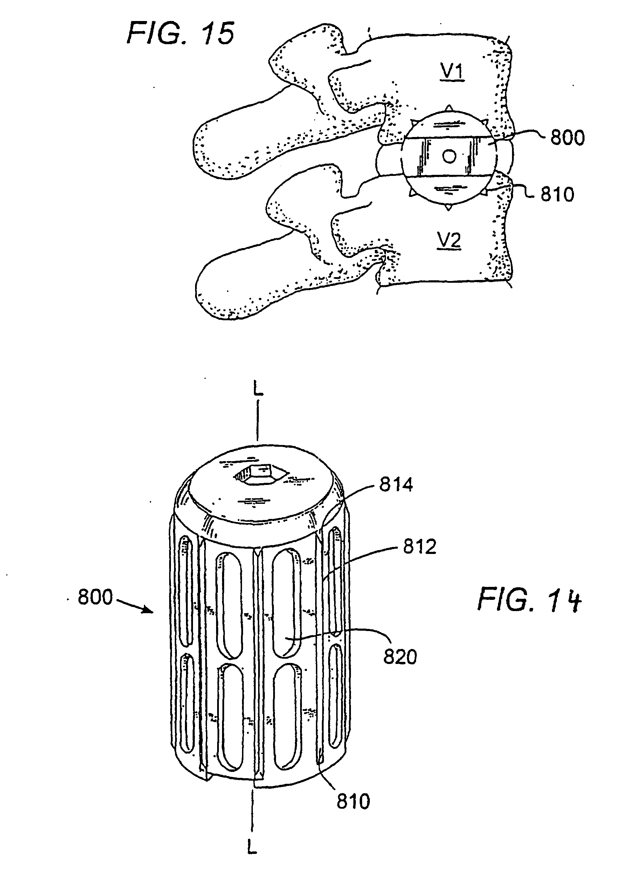

Referring to Figure 14, an alternative embodiment of the spinal fusion implant of the present invention is shown and generally referred to by the numeral 800. The spinal fusion implant 800 is similar in configuration to the spinal fusion implant 100 discussed above. However, instead of an external thread, the spinal fusion implant 800 has a plurality of longitudinal splines 810 along its external surface. The splines 810 are parallel to the central longitudinal axis L of the implant 800 in the direction of insertion of the implant 800. The splines 810 have a sharp edge 812 and a sharpened leading end 814 to facilitate insertion of the spinal fusion implant 800 into the adjacent vertebrae. Located between the splines 812 are a plurality of slots 820 that allow bone growth into the implant and into the internal chamber of the implant 800 during spinal fusion.

Referring to Figure 15, the spinal fusion implant 800 is shown inserted from the lateral aspect of the spine into a bore created across the disc space D and into the adjacent vertebrae V₁ and V₂ along the transverse width of the vertebrae V₁ and V₂. The spinal fusion implant 800 is pushed into place by linear advancement such that the splines 810 engage a portion of each of the adjacent vertebrae V₁ and V₂. The splines 810 function to engage the vertebrae V₁ and V₂ and stabilize the spinal fusion implant 800 once implanted. The splines 810 are oriented longitudinally with respect to the spinal fusion implant 800 to prevent any dislodgement of the spinal fusion implant 800 from between the vertebrae V₁ and V₂ as result of anterior to posterior motion of the spine. It is appreciated that the number of splines 810 and the configuration of the splines 810 can vary depending on the size of the spinal fusion implant 800 being implanted.

Referring to Figure 16, an alternative embodiment of the spinal fusion implant of the present invention is shown and generally referred to by the numeral 900. The spinal fusion implant 900 differs from the implants described above in that it is inserted in the disc space D between the adjacent vertebrae of the spine and not into a cylindrical bore created across the disc space. Therefore, the spinal fusion implant 900 does not require the removal of any portion of bone from the adjacent vertebrae as the spinal fusion implant 900 fits within the natural disc space between the adjacent vertebrae. However, the removal of at least a portion of the disc material present between the adjacent vertebrae is required for proper insertion.

The spinal fusion implant 900 comprises a rectangular block 901 having a top surface 902 and a bottom surface 904 for engaging the adjacent vertebrae and may be flat or may conform at least in part. The top and bottom surfaces 902 and 904 may comprise any of the surface roughenings described herein for engaging the bone of the adjacent vertebrae to promote firm stability. The spinal fusion implant 900 may be solid or hollow at least in part and have a plurality of openings 906 to allow bone ingrowth. The openings 906 may be present on all surfaces of the implant 900 and may either pass through the entire implant 900, or may be closed bottom wells for holding fusion promoting materials.

Referring to Figure 17, the spinal fusion implant 900 is shown implanted from the lateral aspect of the spine in the disc space D between two adjacent vertebrae V, V₁ and V₂ along the transverse width of the adjacent vertebrae V₁ and V₂. The spinal fusion implant 900 has a height that is substantially equal to the height of the disc space D, a length that is greater than one half the transverse width W of the vertebrae and a width that approximates the depth of the vertebrae.

In the preferred embodiment, the spinal fusion implant 900 has a height in the range of 8 mm to 16 mm, with the preferred height being 10-12 mm; a width in the range of 24 mm to 32 mm, with the preferred width being 26 mm; and a length in the range of 32 mm to 50 mm, with 42 mm being the preferred length.

Referring to Figure 18, an alternative embodiment of the spinal fusion implant of the present invention is shown and generally referred to by the numeral 1000. The spinal fusion implant 1000 is similar to the spinal fusion implant 900, but has a narrower width such that more than one spinal fusion implant 1000 may be combined in a modular fashion for insertion within the disc space D between the adjacent vertebrae.

Referring to Figure 19, a plurality of spinal fusion implants 1000 are shown combined in a modular fashion inserted in the disc space D from the lateral aspect of the spine and along the transverse width of the vertebrae V₁ and V₂.

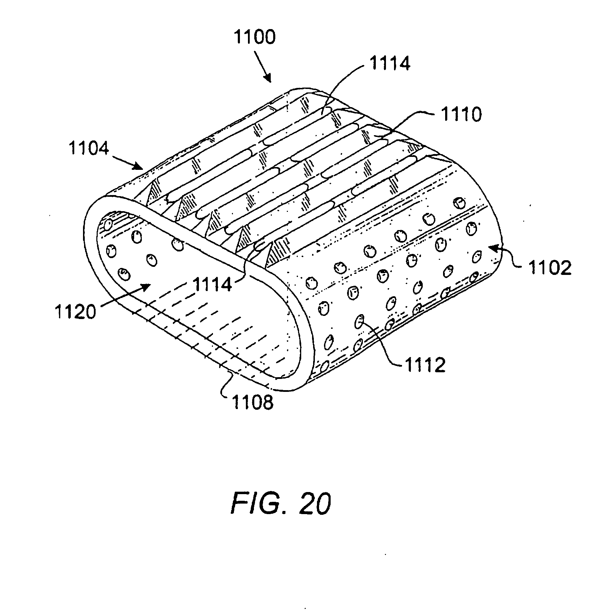

Referring to Figure 20, an alternative embodiment of the spinal fusion implant of the present invention is shown and generally referred to by the numeral 1100. The spinal fusion implant 1100 is inserted into the disc space between two adjacent vertebrae from the lateral aspect of the spine and along the transverse width of the vertebrae. The implant 1100 is dimensioned to replace the natural disc material present between two adjacent vertebrae. The implant 1100 has a generally rectangular body with curved sides 1102 and 1104. The top and bottom surfaces 1106 and 1108 have a plurality of splines 1110 similar in structure and function as the splines 810 described above. As the implant 1100 is inserted in the disc space, the splines 1110 engage the bone of the adjacent vertebrae.

The implant 1100 is shown as being hollow with openings 1112 and slots 1114 in the outer surface of the implant 1100 permitting bone ingrowth into the interior of the implant 1100. However, it is appreciated that the implant 1100 may be solid and may have channels or wells in place of opening 1112 to permit bone ingrowth and incorporation of the implant 1100 into the spinal fusion mass. The interior of the implant 1000 may be accessed through the aperture 1120 which may be closed with a snap fit cover.

## Claims

1. A translateral spinal implant for insertion from the lateral aspect of the spine from a position anterior to the transverse process of the spine and into the disc space between two adjacent vertebrae, said implant having a length that is greater than one half the transverse width of the adjacent vertebrae, said length being substantially greater than the depth of the adjacent vertebrae, and a height at least sufficient to contact each of the adjacent vertebrae and for restoring the height of the disc space.

2. The spinal implant according to claim 1, in which said implant has opposed arcuate portions for engaging each of said adjacent vertebrae, said implant having a diameter in the range of 22 mm to 30 mm.

3. The spinal implant according to one of claims 1-2 in which said implant is made of an artificial material other than bone.

4. The spinal implant according to one of claims 1-2 in which said implant is made of an artificial material stronger than bone.

5. The spinal implant according to one of claims 1-4 in which said implant is capable of penetrably engaging both of the adjacent vertebrae.

6. The spinal implant of according to one of claims 1-4 further comprising surface roughenings for engaging the adjacent vertebrae and for maintaining said implant in place, said surface roughenings being present on at least a portion of the exterior of said implant.

7. The spinal implant of claim 6 in which said surface roughenings include a plurality of splines.

8. The spinal implant according to one of claims 1-4 further comprising an external thread for engaging the vertebrae.

9. The spinal implant according to one of claims 1-8 in which said implant has a plurality of openings capable of retaining fusion promoting material.

10. The spinal implant according to one of claims 1-9 in which said implant comprises a plurality of modular members, each of said modular members having a length that is greater than one half the transverse width of the adjacent vertebrae, said length being substantially greater than the depth of the adjacent vertebrae, and a width less than the depth of the adjacent venebrae.

11. The spinal implant of claim 10 in which each of said modular members comprises upper and lower walls, and side walls, said upper and lower walls forming a support structure including at least a portion of the interior surface of said upper and lower walls for bearing against the adjacent vertebrae.

12. The spinal implant according to one of claims 1-9 in which said implant has a width that is at least as great as the height.

13. The spinal implant according to one of claims 1-12 for use in the lumbar spine in which the length of said implant is in the range of approximately 35 mm to 50 mm.

14. The spinal implant according to one of claims 1-12 for use in the thoracic spine in which the length of said implant is in the range of approximately 12 mm to 30 mm.

15. The spinal implant according to one of claims 1-14 in which said implant has an outer surface with at least one opening passing through said implant, said opening capable of permirting bone growth in continuity from one of said adjacent vertebrae to the other of said two adjacent vertebrae to permit fusion of said two adjacent vertebrae to occur at least in part through said implant.

16. The spinal implant according to one of claims 1, 3-15 having upper and lower arcuate surfaces for contacting the adjacent vertebrae.

17. The spinal implant of claim 16 having a substantially cylindrical configuration.

18. The spinal implant according to one of claims 1-9 and 12-17 in which said implant is modular.

19. The spinal implant according to one of claims 1-4 having a width approximating the depth of the adjacent vertebrae.

20. The implant according to one of claims 1 to 19 in which said implant comprises an osteogenic material.

21. The spinal implant of claim 20 in which said osteogenic material includes at least one of bone morphogenetic protein, harvested bone, hydroxyapatite, and hydroxyapatite tricalcium phosphate.

22. The spinal implant according to one of claims 1 to 19, in which said implant comprises at least in part fusion promoting material.

23. The spinal implant according to claim 22, in which said fusion promoting material includes at least one of bone, bone morphogenetic protein, hydroxyapatite, and hydroxyapatite tricalcium phosphate.

24. The spinal implant according to claim 1, wherein more than one spinal fusion implants are combined in a modular fashion within the disk space between the adjacent vertebrae.

## Patentansprüche

1. Translaterales Spinalimplantat zum Einsetzen von der Seite der Wirbelsäule aus einer Position anterior zum Querfortsatz der Wirbelsäule in den Bandscheibenzwischenraum zwischen zwei benachbarten Wirbeln hinein, wobei das Implantat eine Länge aufweist, die größer ist als die halbe Querausdehnung der benachbarten Wirbel, wobei dessen Länge im Wesentlichen größer ist als die Tiefe der benachbarten Wirbel und dessen Höhe zumindest ausreicht, um jeden der benachbarten Wirbel zu berühren, und zum Wiederherstellen der Höhe des Bandscheibenzwischenraums.

2. Spinalimplantat nach Anspruch 1, wobei das Implantat gegenüberliegende gekrümmte Abschnitte für den Eingriff mit jedem der benachbarten Wirbel aufweist, wobei das Implantat einen Durchmesser zwischen 22 mm und 30 mm hat.

3. Spinalimplantat nach einem der Ansprüche 1 - 2, wobei das Implantat aus einem künstlichen Material und nicht aus Knochen besteht.

4. Spinalimplantat nach einem der Ansprüche 1 - 2, wobei das Implantat aus einem künstlichen Material besteht, das fester als Knochen ist.

5. Spinalimplantat nach einem der Ansprüche 1 - 4, wobei das Implantat imstande ist, beide benachbarten Wirbel durchdringend in Eingriff zu nehmen.

6. Spinalimplantat nach einem der Ansprüche 1 - 4, das weiterhin Oberflächenaufrauungen für den Eingriff mit den benachbarten Wirbeln und für das Festhalten des Implantats aufweist, wobei die Oberflächenaufrauungen wenigstens auf einem Teil der Außenfläche des Implantats vorliegen.

7. Spinatimplantat nach Anspruch 6, wobei die Oberflächenaufrauungen eine Vielzahl von Keilwellen umfassen.

8. Spinalimplantat nach einem der Ansprüche 1 - 4, das weiterhin ein Außengewinde für den Eingriff mit den Wirbeln aufweist.

9. Spinalimplantat nach einem der Ansprüche 1 - 8, wobei das Implantat eine Vielzahl von Öffnungen aufweist, die imstande sind, ein fusionsförderndes Material aufzunehmen.

10. Spinalimplantat nach einem der Ansprüche 1 - 9, wobei das Implantat eine Vielzahl modularer Elemente umfasst, wobei jedes modulare Element eine Länge aufweist, die größer ist als die halbe Querausdehnung der benachbarten Wirbel, wobei dessen Länge im Wesentlichen größer ist als die Tiefe der benachbarten Wirbel und dessen Breite kleiner ist als die Tiefe der benachbarten Wirbel.

11. Spinalimplantat nach Anspruch 10, wobei jedes der modularen Elemente eine obere und eine untere Wand sowie Seitenwände umfasst, wobei die obere und die untere Wand eine Trägerstruktur mit wenigstens einem Teil der Innenfläche der oberen und der unteren Wand zum Abstützen an den benachbarten Wirbeln bilden.

12. Spinalimplantat nach einem der Ansprüche 1 - 9, wobei das Implantat eine Breite hat, die wenigstens so groß ist wie die Höhe.

13. Spinalimplantat nach einem der Ansprüche 1 - 12 zur Anwendung in der Lendenwirbelsäule, wobei die Länge des Implantats etwa zwischen 35 mm und 50 mm beträgt.

14. Spinalimplantat nach einem der Ansprüche 1 - 12 zur Anwendung in der Brustwirbelsäule, wobei die Länge des Implantats etwa zwischen 12 mm und 30 mm beträgt.

15. Spinalimplantat nach einem der Ansprüche 1 - 14, wobei das Implantat eine Außenfläche mit wenigstens einer durch das Implantat verlaufenden Öffnung hat, wobei die Öffnung imstande ist, das Knochenwachstum kontinuierlich von einem der benachbarten Wirbel zum anderen der beiden benachbarten Wirbel zu ermöglichen und damit eine Fusion der beiden benachbarten Wirbel wenigstens teilweise durch das Implantat hindurch zu gestatten.

16. Spinalimplantat nach einem der Ansprüche 1, 3 - 15 mit einer oberen und unteren gekrümmten Oberfläche für den Kontakt mit den benachbarten Wirbeln.

17. Spinalimplantat nach Anspruch 16 mit im Wesentlichen zylindrischem Aufbau.

18. Spinalimplantat nach einem der Ansprüche 1 - 9 und 12 - 17, wobei das Implantat modular ist.

19. Spinalimplantat nach einem der Ansprüche 1 - 4 mit einer Breite, die in etwa der Tiefe der benachbarten Wirbel entspricht.

20. Spinalimplantat nach einem der Ansprüche 1 bis 19, wobei das Implantat ein osteogenetisches Material umfasst.

21. Spinalimplantat nach Anspruch 20, wobei das osteogenetische Material wenigstens eines der Folgenden aufweist: morphogenetisches Knochenprotein, körpereigenen Knochen, Hydroxyapatit und Hydroxyapatit-Tricalciumphosphat.

22. Spinalimplantat nach einem der Ansprüche 1 bis 19, wobei das Implantat wenigstens teilweise fusionsförderndes Material aufweist.

23. Spinalimplantat nach Anspruch 22, wobei das fusionsfördernde Material wenigstens eines der Folgenden umfasst: Knochen, morphogenetisches Knochenprotein, Hydroxyapatit und Hydroxyapatit-Tricalciumphosphat.

24. Spinalimplantat nach Anspruch 1, wobei mehr als ein spinales Fusionsimplantat innerhalb des Bandscheibenzwischenraums zwischen den benachbarten Wirbeln modular miteinander kombiniert werden.

## Revendications

1. Implant vertébral translatéral pour insertion à partir de l'orientation latérale de la colonne à partir d'une position antérieure à l'apophyse transverse de la colonne et dans l'espace discal séparant deux vertèbres adjacentes, ledit implant ayant une longueur supérieure à la moitié de la largeur transversale des vertèbres adjacentes, ladite longueur étant substantiellement supérieure à la profondeur des vertèbres adjacentes, et une hauteur au moins suffisante pour entrer en contact avec chaque vertèbre adjacente et pour rétablir la hauteur de l'espace discal.

2. Implant vertébral selon la revendication 1, dans lequel ledit implant comporte des portions arquées opposées pour venir en contact avec lesdites vertèbres adjacentes, ledit implant ayant un diamètre dans la plage de 22 mm à 30 mm.

3. Implant vertébral selon l'une des revendications 1 et 2, dans lequel ledit implant est constitué par un matériau artificiel différent de l'os.

4. Implant vertébral selon l'une des revendications 1 et 2, dans lequel ledit implant est formé d'un matériau artificiel plus résistant que l'os.

5. Implant vertébral selon l'une des revendications 1 à 4, dans lequel ledit implant est capable de s'engager par pénétration dans les deux vertèbres adjacentes.

6. Implant vertébral selon l'une des revendications 1 à 4 comprenant en outre des rugosités de surface pour venir en contact avec les vertèbres adjacentes et pour maintenir en place ledit implant, lesdites rugosités de surface étant présentes sur au moins une partie de l'extérieur dudit implant.

7. Implant vertébral selon la revendication 6, dans lequel les rugosités de surface comprennent une pluralité de cannelures.

8. Implant vertébral selon l'une des revendications 1 à 4 comprenant en outre un filetage externe pour tenir en contact avec les vertèbres.

9. Implant vertébral selon l'une des revendications 1 à 8, dans lequel ledit implant comprend une pluralité d'ouvertures capables de retenir un matériau promoteur de fusion.

10. Implant vertébral selon l'une des revendications 1 à 9, dans lequel ledit implant comprend une pluralité de membres modulaires, chacun desdits membres modulaires ayant une longueur supérieure à la moitié de la largeur transversale des vertèbres adjacentes, ladite longueur étant substantiellement supérieure à la profondeur des vertèbres adjacentes, et une largueur inférieure à la profondeur des vertèbres adjacentes.

11. Implant vertébral selon la revendication 10, dans lequel chacun desdits membres modulaires comprend des parois supérieure et inférieure et des parois latérales, lesdites parois supérieure et inférieure formant une structure de support incluant au moins une portion de la surface intérieure desdites parois supérieure et inférieure pour s'appuyer contre les vertèbres adjacentes.

12. Implant vertébral selon l'une des revendications 1 à 9, dans lequel ledit implant a une largeur au moins égale à la hauteur.

13. Implant vertébral selon l'une des revendications 1 à 12 pour utilisation dans la colonne lombaire, dans lequel la longueur dudit implant est dans la plage d'environ 35 mm à 50 mm.

14. Implant vertébral selon l'une des revendications 1 à 12 pour utilisation dans la colonne thoracique, dans lequel la longueur dudit implant est dans la plage d'environ 12 mm à 30 mm.

15. Implant vertébral selon l'une des revendications 1 à 14, dans lequel ledit implant possède une surface externe comprenant au moins une ouverture traversant ledit implant, ladite ouverture pouvant permettre une croissance osseuse en continuité de l'une desdites vertèbres adjacentes à l'autre desdites deux vertèbres adjacentes pour permettre qu'une fusion desdites deux vertèbres adjacentes se produise au moins en partie au travers dudit implant.

16. Implant vertébral selon l'une des revendications 1, 3 à 15 pourvu de surfaces arquées supérieure et inférieure pour entrer en contact avec les vertèbres adjacentes.

17. Implant vertébral selon la revendication 16 ayant une configuration substantiellement cylindrique.

18. Implant vertébral selon l'une des revendications 1 à 9 et 12 à 17, dans lequel ledit implant est modulaire.

19. Implant vertébral selon l'une des revendications 1 à 4 ayant une largeur approximativement égale à la profondeur des vertèbres adjacentes.

20. Implant selon l'une des revendications 1 à 19, dans lequel ledit implant comprend un matériau ostéogène.

21. Implant vertébral selon la revendication 20, dans lequel ledit matériau ostéogène inclut l'un au moins parmi une protéine morphogène osseuse, de l'os récolté, de l'hydroxyapatite et du phosphate d'hydroxyapatite tricalcique.

22. Implant vertébral selon l'une des revendications 1 à 19, dans lequel ledit implant comprend au moins en partie un matériau promoteur de fusion.

23. Implant spinal selon la revendication 22, dans lequel ledit matériau promoteur de fusion inclut l'un au moins parmi de l'os, une protéine morphogène osseuse, de l'hydroxyapatite et du phosphate d'hydroxyapatite tricalcique.

24. Implant vertébral selon la revendication 1, dans lequel plus d'un implant de fusion vertébrale sont combines de manière modulaire à l'intérieur de l'espace discal séparant les vertèbres adjacentes.
